# DEMANDE DE BREVET EUROPEEN

(11) **EP 1 493 400 A1**
(43) Date de publication de la demande: **05.01.2005**
(21) Numéro de dépôt: 04291641.1
(22) Date de dépôt: 29.06.2004
(51) Int. Cl.: A61F 2/00

(54) **Dispositif pour incontinence urinaire**

(30) Priorité: 04.07.2003 FR 0308220
(71) Demandeur: Forgeot, Sylviane, 75116 Paris (FR); Aubert, Véronique, 75116 Paris (FR)
(72) Inventeur: Forgeot, Sylviane, 75116 Paris (FR); Aubert, Véronique, 75116 Paris (FR)
(74) Mandataire: Bentz, Jean-Paul

(57) **Abrégé**

L'invention a pour objet un dispositif pour incontinence urinaire de forme sensiblement cylindrique, imperméable, comprenant des fibres de coton recouvertes d'une composition.

Ce dispositif se caractérise en ce que les fibres de coton sont compactées, en ce que les fibres constituant la surface externe du dispositif sont lisses sur toutes leur longueur, et ce que la composition recouvrant en partie ou en totalité les fibres de coton est une composition lubrifiante, imperméabilisant le dispositif, et ayant un effet thérapeutique et/ou hydratant.

## Description

La présente invention se rapporte à un dispositif pour incontinence urinaire.

Certaines femmes, particulièrement celles qui ont données naissance à des enfants ou encore les femmes ménopausées sont sujettent à des fuites urinaires involontaires. De telles fuites liées à l'effort se produisent par exemple, lors de la toux, de rire, de stress, de la pratique de certains sports ou bien encore tout simplement sans raisons apparentes.

Ceci représente alors pour ces femmes un handicap majeur pour la vie quotidienne.

Parmi les techniques possibles connues à ce jour, on peut citer l'intervention chirurgicale qui consiste à mettre en place un fil ou une bandelette sous la partie inférieure de l'urètre pour soutenir ce dernier, et ainsi éviter cet écoulement incontrôlé d'urine.

Malheureusement une telle technique, particulièrement lourde en terme de mise en oeuvre présente aussi l'inconvénient de ne pas toujours résoudre correctement, et totalement, ce problème d'incontinence féminine. En effet, une telle technique qui, le plus souvent est utilisée dans le cas d'incontinence sévère, pose aussi le problème de devoir être renouvelée du fait des mauvais résultats obtenus, avec tous les inconvénients liées à une hospitalisation.

Une autre technique possible connue jusqu'ici pour traiter l'incontinence urinaire féminine consiste à appliquer des impulsions électriques à l'entrée du vagin.

Mais une telle technique s'avère être onéreuse, fastidieuse quant à son utilisation, et ne présente pas forcément tous les résultats escomptés.

Par ailleurs, l'absorption orale de certains médicaments ayant pour rôle de diminuer les contractions de la vessie, entraînent des effets secondaires particulièrement gênants, tels que la sécheresse de la bouche.

Parmi des techniques connues plus simples à mettre en oeuvre, on peut citer celles des documents WO00/37013 et WO02/26160 qui décrivent chacune un dispositif pour incontinence urinaire féminine, imperméable, et constitué de deux éléments distincts constituant à la fois le coeur interne du dispositif, et son enveloppe externe. Le coeur interne constitué d'un membre résistant a pour but d'effectuer un soutien mécanique de l'urètre, alors que l'enveloppe externe est destinée à être en contact direct avec les muqueuses vaginales.

Malgré sa constitution relativement simple, un tel dispositif nécessite l'assemblage de différents éléments. Ce document ne précise pas la présence d'une crème lubrifiante, dont la présence permet notamment l'assouplissement des muqueuses vaginales afin d'éviter leur irritation lors de la mise en place et du retrait du dispositif.

On connaît de plus du document WO00/67662 un dispositif pour incontinence urinaire comprenant au moins un moyen support courbé, souple, destiné au support mécanique de l'urètre, et un moyen absorbant les fuites urinaires.

Or l'absorption des fuites urinaires par un tel dispositif entraîne malgré tout la présence d'odeurs désagréables, plus ou moins prononcées et particulièrement gênantes pour l'utilisatrice et éventuellement pour son entourage proche.

Aussi il subsiste le besoin de disposer d'un dispositif pour incontinence urinaire féminine rigide, de constitution plus simple que ceux connus jusqu'ici, qui permet le soutien mécanique parfait de l'urètre sans subir de déformation importante au cours de la journée de port, qui soit facile à mettre en place et à enlever dans le vagin, qui soit confortable à porter toute la journée, et qui n'absorbe absolument pas les fuites urinaires.

L'invention a donc pour objet un dispositif pour incontinence urinaire de forme sensiblement cylindrique, imperméable, comprenant des fibres de coton recouvertes d'une composition. Le dispositif se caractérise en ce que les fibres constituant la surface externe du dispositif sont lisses sur toutes leur longueur, et en ce que la composition recouvrant en partie ou en totalité les fibres de coton est une composition lubrifiante, imperméabilisant le dispositif et ayant un effet thérapeutique et/ou hydratant.

Le dispositif selon l'invention présente l'avantage d'être de constitution nettement plus simple que les dispositifs similaires existants déjà, d'être étanche à tout liquide provenant de l'organisme, d'avoir un coût de revient relativement faible, et de permettre éventuellement, en plus du soutien mécanique, un soin aisé des muqueuses internes du tractus féminin intérieur du fait de la présence éventuelle de composés à effet thérapeutique.

De préférence, la composition à effet thérapeutique pouvant être présente sur le dispositif comprend un agent choisi parmi les agents estrogéniques, les agents antimicrobiens, les agents anti-inflammatoires, les agents antiviraux et leur mélange.

La composition à effet hydratant peut être choisie parmi les crèmes hydratantes, mais est différente des gels hydratants

Pour un retrait plus simple, le dispositif de l'invention peut comprendre un moyen de retrait (par exemple type cordelette) pouvant éventuellement être fixé à l'intérieur et/ou à l'extérieur des fibres de coton. De même que pour son retrait, le dispositif de l'invention peut comprendre un applicateur, de préférence en matière plastique, destiné à faciliter sa mise en place dans l'organisme.

L'invention va maintenant être décrite à l'aides des exemples ci-dessous basés sur les figures annexées, et dans lesquelles :
- la figure 1 représente une vue schématique en coupe du dispositif selon l'invention,
- la figure 2 représente une vue schématique en coupe du bassin féminin comprenant le dispositif selon l'invention,
- la figure 3 représente une vue schématique en coupe du dispositif de l'invention présenté dans son étui avant sa préparation pour son utilisation,
- la figure 4 représente une vue schématique en coupe du dispositif de l'invention préparé et extrait de son étui avant son utilisation.

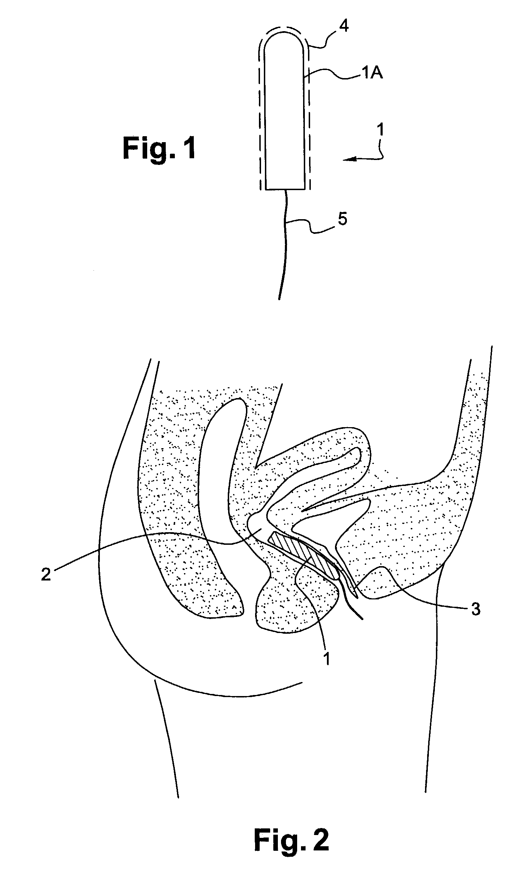

Comme on peut le voir sur la figure 1, le dispositif selon l'invention de référence générale 1 est sensiblement de forme cylindrique. Il est constitué d'un ensemble de fibres de coton compactées, très denses et suffisamment rigide pour pénétrer dans l'entrée du vagin sans subir de quelconque déformation lors de la pénétration, et lors du support mécanique quotidien. Le dispositif 1 peut présenter une longueur comprise entre 4 et 6cm, et de préférence de 5cm, et un diamètre compris entre 1 et 1,5cm, et de préférence 1cm.

Le dispositif selon l'invention peut exister en différentes tailles. La plus petite taille (environ 5cm) peut comprendre moins de composition lubrifiante que la plus grande, afin de mieux s'adapter à l'organisme de différentes utilisatrices.

Les fibres constituant la partie extérieures 1A du dispositif sont parfaitement lisses, et ne présentent aucune rainures entre elles de manière à parfaire l'étanchéité vis-à-vis des fuites urinaires.

Comme on peut le voir sur la figure 2, et du fait de sa constitution uniquement à base de fibres de coton, le dispositif 1 de l'invention, une fois disposé dans le vagin 2, épousera facilement et rapidement, et avec l'aide de la chaleur corporel, le volume dans lequel il est disposé.

Ainsi, du fait de la présence du dispositif 1, l'urètre 3 est donc remonté mécaniquement vers le haut du bassin. Par cette action de remontée supérieure de l'urètre, le dispositif 1 de l'invention effectue la fermeture de la sortie de l'urètre, et évite donc toute fuite urinaire vers l'extérieur.

La présence d'une composition 4 qui revêtit tout ou partie du dispositif 1 l'imperméabilise contre toute absorption éventuelle d'urine. Ceci lui permet de rester propre et de ne présenter aucune odeur désagréable sur tout le long de la journée de port.

Par ailleurs, la composition 4, outre son effet imperméabilisant permet d'assurer l'étanchéité parfaite de la fermeture de l'urètre.

Enfin, la présence de cette composition 4 sur les fibres extérieures du dispositif 1 facilite la pénétration ou le retrait du dispositif dans le vagin, et ceci même plusieurs fois au cours de la journée sans risque d'irritation et/ou de douleur liés à ces opérations.

En effet, le dispositif 1 de l'invention sera de préférence enlevé à chaque fois que l'utilisatrice souhaitera uriner afin d'éviter toute souillure inutile du dispositif, et par conséquent sera remis en place une fois l'évacuation d'urine effectuée.

Le dispositif de l'invention est de préférence disposé dans le vagin le matin lors de la toilette matinale, et enlevé définitivement le soir avant le coucher. L'intérêt d'un tel dispositif est qu'il permet d'effectuer le maintien mécanique de l'urètre quotidien, avec l'emploi d'un unique dispositif selon l'invention par jour puisqu'il n'est pas souillé au cours de la journée.

Pendant le port quotidien du dispositif de l'invention, le cycle urinaire n'est absolument pas perturbé.

La présence de la composition 4 sur des fibres extérieures parfaitement lisses permet de combiner une étanchéité parfaite du dispositif par rapport aux fuites urinaires ainsi qu'un modelage parfait du dispositif sur les muqueuses vaginales.

Pour des femmes ayant encore leur règles menstruelles, le dispositif 1 de l'invention ne pourra pas être utilisé pendant les quelques jours de règles puisqu'il n'absorbe aucun liquide. Il pourra alors être remplacé par les protections classiques connues à cet effet.

Le dispositif de l'invention peut comprendre par ailleurs un moyen de retrait 5, de type cordelette, permettant son retrait facile de l'organisme. Le moyen de retrait peut être fixé par tous moyens connus de fixation dans et/ou autour les fibres de coton compactées afin qu'il ne s'en détache pas au cours de la journée du fait des différents réguliers du dispositif de l'organisme pour uriner.

Le dispositif selon l'invention peut se présenter dans un étui individuel habituellement utilisé, comme par exemple une matière plastique ou métallique, tel que l'aluminium. Il peut comprendre par ailleurs dans le même emballage, et selon différentes variantes de présentation, un applicateur destiné à sa mise en place dans l'organisme.

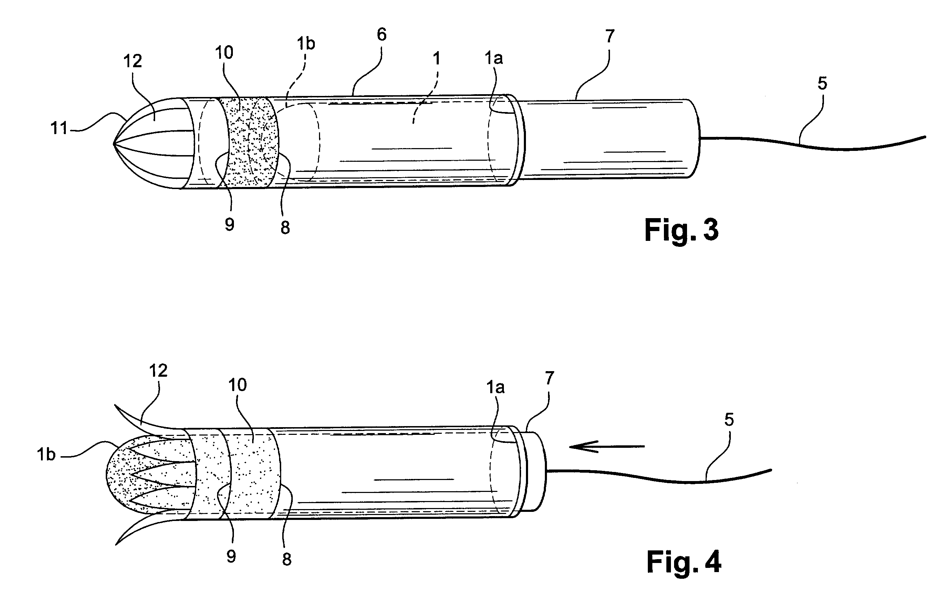

Comme le montre la figure 3, le dispositif 1 de l'invention est disposé dans un étui individuel 6. L'extrémité 1a du dispositif 1 est fixée de manière amovible à un moyen poussoir 7 rigide, par exemple constitué en matière plastique.

L'extrémité la du dispositif comprend un moyen de retrait 5. Dans cet étui 6, l'extrémité 1b sensiblement arrondie du dispositif 1 est disposée en butée contre un premier moyen 8 de retenu de type anneau, par exemple en matière plastique étanche aux microbes.

L'étui 6 comprend en outre un second moyen 9 de retenu, également de type anneau, par exemple en matière plastique étanche aux microbes.

Les moyens 8 et 9 délimitent un volume 10 stérile comprenant la composition destinée à enrober le dispositif 1 de l'invention.

L'extrémité terminale 11 de l'étui 6 comprend un moyen 12 de fermeture, ayant par exemple la forme d'une corolle fermée.

La figure 4, qui représente le dispositif 1 de l'invention extrait de son étui 6, montre le moyen 12 en position ouverte. Sur cette figure, le dispositif 1 est déjà imprégné par la composition présente dans le volume 10.

L'extraction du dispositif 1 de l'invention de son étui 6 s'effectue de la manière suivante.

On exerce avec la main ou avec un simple doigt une force sur le moyen poussoir 7. Cette force permet de percer le moyen de retenu 8 de manière à ce que l'extrémité 1b du dispositif 1 de l'invention soit en contact avec la composition présente dans le volume 10.

L'application continue de la force sur la moyen poussoir 7 permet ensuite à l'extrémité 1b de percer le second moyen de retenu 9 et d'imprégner alors, lors du passage du dispositif dans ce volume 10, toute la surface externe du dispositif 1.

L'extrémité 1b permet, sous l'effet de la pression exercée par le biais du poussoir 7, l'ouverture du moyen de fermeture 12 de manière à extraire le dispositif 1, recouvert sur toute sa surface par la composition présente initialement dans le volume 10.

Le dispositif est alors disponible pour être utilisé dans l'organisme.

Parmi les compositions pouvant être utilisées avec le dispositif de l'invention, on peut en citer quelques exemples qui sont donnés uniquement à titre d'illustration.

### Exemple 1 : Composition se présentant sous forme d'une crème destinée à traiter des affections de la vulve et du vagin liée à la ménopause.

- Estrogènes locaux
- Au moins un excipient choisi parmi la vaseline, des cires émulsionnables, de la paraffine liquide, de la glycérine, des colorants, des conservateurs, de l'eau purifiée.

Cette composition se présente sous forme d'une crème.

Lorsque le dispositif de l'invention est imprégné par cette composition, il peut effectuer à la fois un maintient mécanique de l'urètre ainsi qu'une diffusion au sein des muqueuses vaginales des principes actifs de type estrogène.

### Exemple 2 : Composition se présentant sous forme d'une crème destinée à traiter des dermatophytoses de type intertrigos génital

- Antifongique imidazolé,
- Au moins un excipient choisi parmi des esters polyglycoliques d'acides gras, du propylèneglycol, de la lanoline hydrogénée, des huiles végétales, de la paraffine liquide légère, des conservateurs, des colorants, de l'eau purifiée.

Lorsque le dispositif de l'invention est imprégné par cette composition, il peut effectuer à la fois un maintient mécanique de l'urètre ainsi qu'une diffusion au sein des muqueuses vaginales des principes actifs de type antifongique.

### Exemple 3 : Composition se présentant sous la forme d'une crème destinée à traiter des dermatoses corticosensibes non suintantes.

- dermatocorticoïde
- antibiotique
- au moins un excipient choisi parmi la paraffine liquide, le polyéthylène.

Lorsque le dispositif de l'invention est imprégné par cette composition, il peut effectuer à la fois un maintient mécanique de l'urètre ainsi qu'une diffusion au sein des muqueuses vaginales des principes actifs de type corticoïde.

### Exemple 4 : Composition se présentant sous forme de crème destinée à traiter des vaginites

- anti-infectieux local,
- antifongique à activité anticandida,
- au moins un excipient choisi parmi les glycérides d'huile de palme, de l'huile de soja hydrogénée.

Lorsque le dispositif de l'invention est imprégné par cette composition, il peut effectuer à la fois un maintient mécanique de l'urètre ainsi qu'une diffusion au sein des muqueuses vaginales des principes actifs de type anti-infectieux combinés à un antifongique.

## Revendications

1. Dispositif pour incontinence urinaire de forme sensiblement cylindrique, imperméable, comprenant des fibres de coton recouvertes d'une composition, **caractérisé en ce que** les dites fibres sont compactées, **en ce que** les fibres constituant la surface externe du dispositif sont lisses sur toutes leur longueur, et **en ce que** la composition recouvrant en partie ou en totalité les fibres de coton, est une composition lubrifiante, imperméabilisant ledit dispositif, et ayant un effet thérapeutique et/ou hydratant.

2. Dispositif selon la revendication 1, **caractérisé en ce que** la composition à effet thérapeutique comprend un agent choisi parmi les agents estrogéniques, les agents antimicrobiens, les agents anti-inflammatoires, les agents anti-viral et leur mélange.

3. Dispositif selon la revendication 1, **caractérisé en ce que** la composition à effet hydratant est choisie parmi les crèmes hydratantes.

4. Dispositif selon la revendication 1, **caractérisé en ce qu'**il comprend en outre un moyen de retrait, ledit moyen étant fixé à l'intérieur et/ou à l'extérieur des fibres de coton.

5. Dispositif selon la revendication 1, **caractérisé en ce qu'**il comprend en outre un applicateur destiné à faciliter sa mise en place dans l'organisme.

6. Dispositif selon la revendication 1, **caractérisé en ce que** sa longueur est comprise entre environ 4 et 6cm, et de préférence d'environ 5 cm.

7. Dispositif selon la revendication 1, **caractérisé en ce que** son diamètre est compris entre 1 et 1, 5 cm, et de préférence d'environ 1 cm.
